(19) Europäisches Patentamt
European Patent Office
Office européen des brevets

(11) Veröffentlichungsnummer: **0 249 102**
**B1**

(12) # EUROPÄISCHE PATENTSCHRIFT

(45) Veröffentlichungstag der Patentschrift: **08.11.89**

(51) Int. Cl.4: **B01J 47/00**, B01J 31/08, C08F 8/34, C07C 37/20, C08J 5/22

(21) Anmeldenummer: **87107785.5**

(22) Anmeldetag: **29.05.87**

(54) **Mit Mercaptoaminen modifizierte Ionenaustauscher.**

(30) Priorität: **10.06.86 DE 3619450**

(43) Veröffentlichungstag der Anmeldung:
**16.12.87 Patentblatt 87/51**

(45) Bekanntmachung des Hinweises auf die Patenterteilung:
**08.11.89 Patentblatt 89/45**

(84) Benannte Vertragsstaaten:
**BE DE FR GB IT NL**

(56) Entgegenhaltungen:
**EP-A- 0 023 325**
**EP-A- 0 171 758**
**FR-A- 2 319 420**
**US-A- 3 394 089**
**US-A- 4 346 247**

(73) Patentinhaber: **BAYER AG,**
**D-5090 Leverkusen 1 Bayerwerk(DE)**

(72) Erfinder: **Rudolph, Udo, Dr., Scheiblerstrasse 101,**
**D-4150 Krefeld 1(DE)**
Erfinder: **Bachem, Norbert, Dr., Jentgesallee 43,**
**D-4150 Krefeld 1(DE)**
Erfinder: **Wulff, Claus, Dr., Richard-Strauss-Strasse 21,**
**D-4150 Krefeld 1(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Kondensation von Phenolen und Carbonylverbindungen zur Bildung von Bisphenolen ist bekannt. Bei dieser Reaktion wurden bereits die verschiedensten Katalysatoren verwendet, beispielsweise Chlorwasserstoffssäure (US-PS 2 182 308 und 2 191 831), Bortrifluorid (Chemical Abstracts 58, 3338 c), Perchlorsäure (Chemical Abstracts 60, 1626 h), Benzolsulfonsäure (Chemical Abstracts 59, 511 h) und die verschiedensten Kationenaustauschharze (z.B. GB-A 842 209, 849 565 und 883 391). Auch der Zusatz von S-haltigen Verbindungen zum Katalysator ist bekannt, z.B. aus der US-A 2468 982, 2 623 908 die Verwendung von Thioglykolsäure und 3-Mercaptopropionsäure, aus der US-A 2 359 242 der Zusatz von Thiophenolen, aus der US-A 2 775 620 der Zusatz von Alkylmercaptanen, aus Chemical Abstracts 58, 1403 e der Zusatz von Schwefelwasserstoff.

Diese bekannten, S-haltigen Katalysatoren führen in der betrieblichen Praxis zu erheblichen Korrosionsschäden. Die mit diesen Katalysatoren hergestellten Bisphenole sind verunreinigt, Rohprodukte die neben Bisphenol nicht umgesetzes Phenol, Carbonylverbindung, Reaktionswasser und noch unerwünschte Nebenprodukte enthalten, z.B. bei der Bisphenol A Synthese Isomere des Bisphenol A, insbesondere 2.2-(2,4'-Dihydroxidiphenyl)-propan und 2.2(2.2'Dihydroxidiphenyl)-propan, komplexe Produkte, wie das sogenannte "Codimere" 2.2.4-Trimethyl-4-p-hydroxiphenylchroman, Kondensationsprodukte, wie Trisphenol oder noch höher kondensierte Produkte in Form teeriger, auch hochsiedender Substanzen sowie Zersetzungsprodukte und schwefelhaltige Substanzen. Die Anwesenheit dieser Nebenprodukte zu verbleiben und Verfärbungen hervor zurufen. Dies beeinträchtigt die Verwertbarkeit des Endprodukts auch in Fällen, in denen keine besonders hohe Reinheit erforderlich ist. Darüberhinaus verhindern die anwesenden Nebenprodukte einige der üblichen Reaktionen des Bisphenols, insbesondere die weitere Umsetzung zu Polycarbonaten.

Die US-A 3 394 089 beschreibt ein Verfahren zur Herstellung von Bisphenol A aus Aceton und Phenol unter Verwendung eines sulfonsäuregruppenhaltigen Katalysators, dessen Sulfonsäuregruppen zu 5-25 mol% mit Mercaptoaminen unter Ammonsalzbildung blockiert sind, das diese Nachteile vermeiden soll. Dieses modifizierte Ionenaustauscherharz, das durch Neutralisation mit z.B. ß-Mercaptoethylamin in wäßriger Lösung erhalten wird, ist für die Produktion ungeeignet, weil es instabil ist und weil die Mercaptoverbindung im Dauerversuch durch das Reaktionsmedium ausgewaschen wird und der Katalysator dadurch seine Wirkung verliert.

Es wurde nun gefunden, daß hochreine Bisphenole hergestellt werden können, wenn stark saure Ionenaustauscher verwendet werden, die einen sehr hohen bis quantitativen Neutralisationsgrad der Säuregruppen aufweisen und die Neutralisation mit vorher getrocknetem Ionenaustauscher in wasserfreiem Medium vorgenommen wird.

Gegenstand der Erfindung ist somit ein Verfahren zur Herstellung von mit Mercaptoaminen modifizierten trockenen Ionenaustauschern, das dadurch gekennzeichnet ist, daß man bekannte saure Ionenaustauscherharze, mit Totalkapazitäten an Säurefunktionen in wasserfeuchter Form mit einem Wassergehalt von ca. 75 bis 85 Gew.-%, von 0,7 bis 2,1 mval/ml Ionenaustauscher oder mit Totalkapazitäten an Säurefunktionen von 3,5 bis 5 mval oder höher, bezogen auf 1 g Trockensubstanz, an Ionenaustauscher, trocknet und danach mit dem für die Bisphenolherstellung erforderlichen Phenol spült und danach oberhalb des Schmelzpunktes dieses Phenols mit mindestens 0,3 Molen, vorzugsweise mit 0,4 Molen bis 1 Mol, pro Mol Säurefunktion der Ionenaustauscher, an Mercaptoaminen der Formel (I) neutralisiert.

Gegenstand der vorliegenden Erfindung sind außerdem die nach dem erfindungsgemäßen Verfahren erhältlichen, mit Mercaptoaminen der Formel (I) modifizierten Ionenaustauscher.

Die Säurefunktionen dieser modifizierten Ionenaustauscher sind zu mindestens 30 Mol-%, vorzugsweise von 40 Mol-% bis 100 Mol-% mit dem Mercaptoamin der Formel (I) belegt.

Geeignete saure Ionenaustauscher sind z.B. übliche Umsetzungsprodukte von Styrol-Divinylbenzol-Copolymerisaten mit üblichen Sulfonierungsmitteln, wie Schwefelsäure, Chlorsulfonsäure u.a., die in Kugelform vorliegen können und die Korngrößen von 0,3 -1,5 mm Durchmesser aufweisen. Sie können vom Geltyp oder makroporös sein. Ihre Totalkapazität an Säurefunktionen in wasserfeuchter Form mit einem Wassergehalt von mit ca. 75 bis 85 Gew.% reicht von 0,7 bis 2,1 mval/ml Ionenaustauscher oder von 3,5 bis 5 mval oder höher, bezogen auf 1 g Trockensubstanz an Ionenaustauscher.

Vor der Behandlung beziehungsweise Neutralisation mit den erfindungsgemäß zu verwendenden Mercaptoaminen der Formel (I), werden diese Ionenaustauscher gegebenenfalls durch Wärme, gegebenenfalls im Vakuum oder gegebenenfalls durch Waschen mit hydrophilen organischen Flüssigkeiten wie Alkoholen oder Phenolen, oder durch azeotrope Destillation mit organischen Flüssigkeiten wie Toluol, Xylol, Methylenchlorid u.a. getrocknet. Danach wird das Ionenaustauscherharz mit dem für die Bisphenolherstellung erforderlichen Phenol gespült und in diesem Medium bei Temperaturen oberhalb des Schmelzpunktes dieses Phenols mit der gewünschten Menge Mercaptoamin der Formel (I) unter Rühren oder in Wirbelschicht versetzt.

In den Mertcaptoaminen der Formel (I)

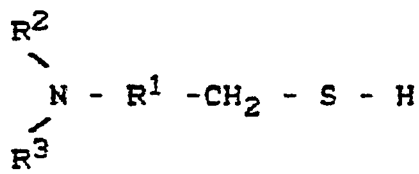

(I),

steht $R^1$ für ein $C_1$-$C_6$-Alkylen, $C_5$-$C_{10}$ Cycloalkylen, $C_6$-$C_{14}$-Arylen, bevorzugt für eine Methylengruppe, und sind $R^2$ und $R^3$ unabhängig voneinander $C_1$-$C_6$ Alkylgruppen oder bevorzugt Wasserstoff. Geeignete Merkaptoamine der Formel (I) sind Dimethylmercaptoethylamin, Ethyl-Cyclohexylmercaptobutylamin, Mercaptopropylamin, 1-Amino-4-mercaptomethylbenzol, ß-Mercaptoethylamin, vorzugsweise ß-Mercaptoethylamin.

Der Einsatz der Mercaptoamine der Formel (I) kann als solcher oder durch in situ Herstellung aus Thiazolidinen wie beispielsweise 2,2-Di-methyldiazolidin erfolgen.

Das durch Neutralisation modifizierte Kationenaustauscherharz kann zur Herstellung zahlreicher Bisphenole aus Phenolen und Carbonylverbindungen eingesetzt werden. Geeignete Phenole sind solche der Formel (II)

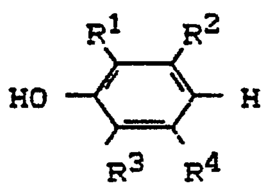

(II)

in welcher

$R^1$, $R^2$, $R^3$ und $R^4$ unabhängig voneinander für Wasserstoff (H), $C_1$-$C_4$-Alkyl oder Halogen wie F, Cl oder Br stehen.

Beispielsweise seien genannt 2.6-Dimethylphenol, o- und m-Kresol, o-sek, Butylphenol, o-tert, Butylphenol, 1,3,5 Xylenol, 2,6-Di-Tert.-butylphenol, Tetrmethylphenol, 2-Methyl-6-tert.-butylphenol, o-Phenylphenol, o- und m-Chlorphenol, o-Bromphenol, 6-Chlor-o-Kresol und 2.6-Dichlorphenol.

Bevorzugt ist unsubstituiertes Phneol.

Geeignete Carbonylverbindungen sind solche der Formel (III)

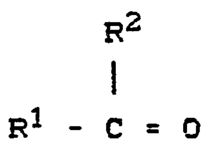

(III),

in welcher

$R^1$ und $R^2$ unabhängig voneinander Wasserstoff (H), $C_1$-$C_6$-Alkyl-, $C_6$-$X_{10}$-Cycloalkyl, $C_6$-$X_{14}$ Aryl, $C_7$-$C_{20}$-Aralkyl, $C_7$-$C_{20}$ Alkylaryl bedeuten oder $R^1$ und $R^2$ gemeinsam einen gesättigten Ring mit 5 bis 6-Ringatomen bilden.

Geeignete Carbonylverbindungen wie Aldehyde und Ketone, sind beispielsweise Formaldehyd, Methylethylketon, Methylpropylketon, Diethylketon, Cyclohexanon, Acetophenon etc. Bevorzugt wird Aceton verwendet.

Die Herstellung kann kontinuierlich oder diskontinuierlich durchgeführt werden, wobei die Verfahrensweise und die erforderlichen Apparate bekannt sind.

Bei diskontinuierlicher Fahrweise werden pro Mol Carbonylverbindungen 80 bis 200 g, vorzugsweise 100 bis 150 g Trockensubstanz an erfindungsgemäßem Ionenaustauscherharz eingesetzt.

Die Reaktionstemperatur bei der Herstellung der Bisphenole liegt im Bereich von 40 - 120°C, vorzugsweise oberhalb des Erstarrungspunktes der beteiligten Komponenten.

Die Reaktions- bzw. Verweilzeit wird so gewählt, daß ein vollständiger Umsatz der verwendeten Carbonylverbindung erzielt wird. Sie beträgt vorzugsweise 30 bis 240 Minuten.

Gegenstand der vorliegenden Erfindung ist somit auch die Verwendung der erfindungsgemäß erhältlichen mit Mercaptoaminen der Formel (I) modifizierten Ionenaustauscher zur Herstellung von Bisphenolen aus Phenolen und Carbonylverbindungen.

Das nach der Umsetzung von Phenol und Carbonylverbindung erhaltene Reaktionsgemisch wird nach üblichen Methoden wie Destillation, Kristallisation etc., aufgearbeitet.

So kann z.B. das hergestellte Bisphenol in der Reaktionsmischung bis zur beginnenden Kristallisation abgekühlt und aus den abfiltrierten Bisphenol-Phenol-Mischkristallen das Phenol durch Destillation oder Extraktion entfernt werden.

Das nach diesem Verfahren hergestellte Bisphenol kann ohne Nachreinigung für bekannte Anwendungsgebiete eingesetzt werden und eignet sich auch für besonders anspruchsvolle Anwendungen wie der Herstellung optisch hochreiner Polycarbonate.

Beispiel 1

Herstellung des modifizierten Ionenaustauscherharzes

Der wasserfeuchte Ionenaustauscher mit ca. 80 Gew.% Feuchte und einer Totalkapazität von 0,75 mval/ml in Lieferform wird zunächst mit destilliertem Wasser gewaschen. Anschließend wird das Harz bei 90 - 100°C und Wasserstrahlpumpenvakuum 24 h lang getrocknet, so daß der Wassergehalt auf < 1 Gew.% fällt.

Das Restwasser wird im azeotropen Gemisch mit Toluol abdestilliert und danach das dem Ionenaustauscherharz noch anhaftende Toluol bei 95°C im Wasserstrahlpumpenvakuum abdestilliert.

120 g des so vorbehandelten Ionenaustauscherharzes werden in einer Rührapparatur in 1128 g Phenol aufgenommen und 24 h unter Feuchtigkeitsauschluß bei 65°C gequollen. Danach wird unter Rühren die für eine bestimmte Belegung in mol% der Beispiele 2 - 10 erforderliche Menge Mercaptoethylamin zugegeben.

Beispiele 2 - 10

Herstellung von Bisphenol A (BPA)

Mit dem unter Beispiel 1 beschriebenen Verfahren wurde das Ionenaustauscherharz mit ß-Mercaptoethylamin so modifiziert, daß in neun Einstellunge 15 - 100% der Sulfonsäuregruppen neutralisiert wurden. Zu der in Beispiel 1 bereiteten Lösung wurden jeweils 58 g Aceton bei 65°C gegeben und nach vollständigem Acetonumsatz die gaschromatographische Reinheit des Bisphenols bzw. die Menge der Nebenkomponenten bestimmt. Die folgende Tabelle faßt die Ergebnisse in Form der Mittelwerte aus je 5 Versuchen/Beispiel zusammen.

| Beispiel | Belegung mol% | GC-Flächenprozente | |
|---|---|---|---|
| | | BPA | Nebenprodukte |
| 2 | 15 | 93,6 | 6,4 (Vergleich) |
| 3 | 20 | 93,8 | 6,2 (Vergleich) |
| 4 | 25 | 94,3 | 5,7 (Vergleich) |
| 5 | 30 | 94,5 | 5,5 |
| 6 | 40 | 94,7 | 5,3 |
| 7 | 50 | 95,1 | 4,9 |
| 8 | 60 | 95,7 | 4,3 |
| 9 | 80 | 96,2 | 3,8 |
| 10 | 100 | 96,7 | 3,3 |

## Patentansprüche

1. Verfahren zur Herstellung von mit Mercaptoaminen modifizierten, trockenen Ionenaustauschern, dadurch gekennzeichnet, daß man bekannte saure Ionenaustauscherharze, mit Totalkapazitäten an Säurefunktionen in wasserfeuchter Form mit einem Wassergehalt von ca. 75 bis 85 Gew.-%, von 0,7 bis 2,1 mval/ml Ionenaustauscher oder mit Totalkapazitäten an Säurefunktionen von 3,5 bis 5 mval oder höher, bezogen auf 1 g Trockensubstanz an Ionenaustauscher, trocknet und danach mit dem für die Bisphenolherstellung erforderlichen Phenol spült und danach oberhalb des Schmelzpunktes dieses Phenols mit mindestens 0,3 Molen, proMol Säurenfunktion des Ionenaustauschers, an Mercaptoaminen der Formel (I) neutralisert

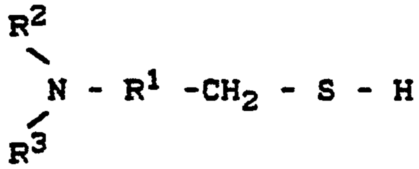 (I),

worin $R^1$ für $C_1$-$C_6$-Alkylen, $C_5$-$C_{10}$-Cycloalkylen und $C_6$-$C_{14}$-Arylen steht und

$R^2$ und $R^3$ unabhängig voneinander $C_1$-$C_6$-Alkylgruppen oder Wasserstoff sind.

2. Mit Mercaptoaminen modifizierte Ionenaustauscher, erhältlich gemäß Anspruch 1.

3. Verwendung der Ionenaustauscher der Anspruchs 2 zur Herstellung von Bisphenolen aus Phenolen und Carbonylverbindungen.

## Claims

1. A process for the preparation of anhydrous ion exchangers modified with mercapto amines, characterised in that known acid ion exchanger resins having total capacities of acid functions of from 0.7 to 2.1 mval/ml of ion exchanger when moist with a water content of about 75 to 85% by weight or total capacities of acid functions of from 3.5 to 5 mval or higher, based on 1 g of the dry substance of ion exchanger, are dried and then rinsed with the phenol required for the preparation of the bisphenol and thereafter neutralized above the melting point of this phenol with at least 0.3 mol of mercapto amines corresponding to formula (I) per mol of acid function of the ion exchanger

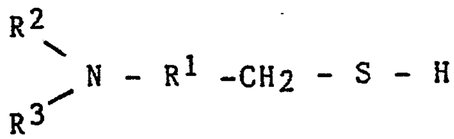 (I)

wherein $R^1$ denotes a $C_1$ to $C_6$ alkylene, $C_5$ to $C_{10}$ cycloalkylene or $C_6$ to $C_{14}$ arylene group and
$R^2$ and $R^3$ denote, independently of one another, $C_1$ to $C_6$ alkyl groups or hydrogen.

2. Ion exchangers modified with mercapto amines obtainable according to Claim 1.

3. Use of the ion exchangers of Claim 2 for the preparation of bisphenols from phenols and carbonyl compounds.

## Revendications

1. Procédé pour la fabrication d'échangeurs d'ions secs modifiés par des mercaptoamines, caractérisé en ce que l'on sèche des résines échangeuses d'ions acides connues ayant des capacités totales en fonctions acides de 0,7 à 2,1 mval/ml d'échangeur d'ions sous forme imbibée d'eau à une teneur en eau d'environ 75 à 85% en poids, ou ayant des capacités totales en fonctions acides de 3,5 à 5 mval ou plus, rapportées à 1 g de substance sèche de l'échangeur d'ions, et on les rince ensuite avec le phénol nécessaire pour la fabrication du bisphénol et ensuite on les neutralise au-dessus du point de fusion de ce phénol avec au moins 0,3 mol par mole de fonctions acides de l'échangeur d'ions, de mercaptoamines de formule (I)

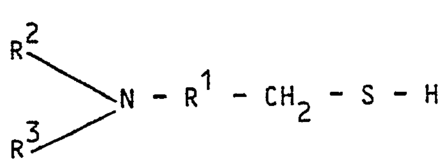 (I)

dans laquelle $R^1$ représente un groupe alkylène en $C_1$–$C_6$, cycloalkylène en $C_5$–$C_{10}$ ou arylène en $C_6$–$C_{14}$ et
$R^2$ et $R^3$ représentent indépendamment l'un de l'autre des groupes alkyles en $C_1$–$C_6$ ou l'hydrogène.

2. Echangeurs d'ions modifiés par des mercaptoamines, que l'on peut obtenir selon la revendication 1.

3. Utilisation des échangeurs d'ions selon la revendication 2 pour la fabrication de bisphénols à partir de phénols et de composés carbonylés.